(19) Europäisches Patentamt
European Patent Office
Office européen des brevets

(11) EP 1 160 243 A1

(12) EUROPEAN PATENT APPLICATION
published in accordance with Art. 158(3) EPC

(43) Date of publication:
05.12.2001 Bulletin 2001/49

(21) Application number: 00906739.8

(22) Date of filing: 06.03.2000

(51) Int Cl.⁷: C07D 239/96

(86) International application number:
PCT/JP00/01360

(87) International publication number:
WO 00/53584 (14.09.2000 Gazette 2000/37)

(84) Designated Contracting States:
AT BE CH CY DE DK ES FI FR GB GR IE IT LI LU MC NL PT SE
Designated Extension States:
AL LT LV MK RO SI

(30) Priority: 09.03.1999 JP 6139099

(71) Applicant: FUJISAWA PHARMACEUTICAL CO., LTD.
Osaka-shi Osaka 541-8514 (JP)

(72) Inventors:
- FUKAGAWA, Masayasu
  Nakaniikawa-gun, Toyama 930-0365 (JP)
- IEDA, Shigeru
  Sanda-shi, Hyogo 669-1544 (JP)
- TSUBOI, Hiroyuki
  Neyagawa-shi, Osaka 572-0024 (JP)
- GOTO, Shunsuke
  Osaka 559-0033 (JP)
- KAGARA, Kooji
  Mino-shi, Osaka 562-0032 (JP)
- UEMATSU, Ryoichi
  Takarazuka-shi, Hyogo 665-0043 (JP)
- NISIWAKI, Masanori
  Yawata-shi, Kyoto 614-8334 (JP)

(74) Representative:
Gille Hrabal Struck Neidlein Prop Roos
Patentanwälte,
Brucknerstrasse 20
40593 Düsseldorf (DE)

# (54) PROCESS FOR PRODUCING QUINAZOLINE DERIVATIVE OR SALT THEREOF

(57) This invention provides a novel industrial process for preparation of a quinazoline derivative (I) represented by the general formula:

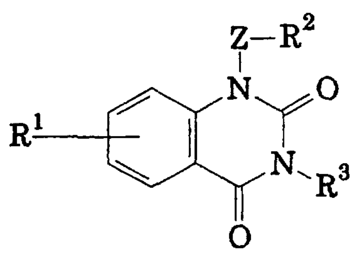

( I )

in which $R^1$ is hydrogen or halogen,

$R^2$ is protected carboxy,
$R^3$ is ar(lower)alkyl which may have one or more suitable substituents,
Z is lower alkylene,

or a salt thereof, by reacting a compound represented by the general formula:

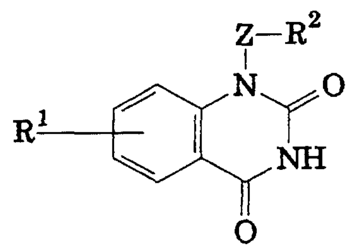

(II)

in which $R^1$, $R^2$ and Z are each as defined above, or a salt thereof, with a compound represented by the general formula:

$$R^3\text{-X} \qquad \text{(III)}$$

in which $R^3$ is as defined above,
X is an acid residue,
or a salt thereof in a suitable solvent in the presence of alkali metal carbonate and further a small amount of water as necessary.

EP 1 160 243 A1

## Description

FIELD OF THE INVENTION

This invention relates to a novel process for preparation of a known quinazoline derivative (I) useful as an intermediate for producing medicaments for the treatment and prevention of diabetic complication and the like, for example.

BACKGROUND ART

A process for preparation of the quinazoline derivative (I) by using a quinazoline derivative (II) as a material compound has been disclosed specifically in Japanese Laid-open Patent Application No. Sho 62-96476, more particularly in Example 4 thereof.

However, this process for preparation uses sodium hydride as a base and is unsuited for mass production in safety.

INDUSTRIAL APPLICABILITY

This invention provides a novel industrial process for preparation of the quinazoline derivative (I).

DISCLOSURE OF THE INVENTION

This invention relates to a process for preparation of the quinazoline derivative (I) represented by the following general formula:

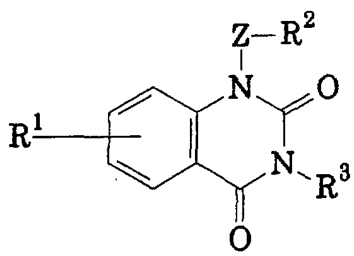

(I)

in which $R^1$ is hydrogen or halogen,

$R^2$ is protected carboxy,
$R^3$ is ar(lower)alkyl which may have one or more suitable substituents,
Z is lower alkylene,

or a salt thereof.

The process for preparation of the quinazoline derivative (I) or a salt thereof in accordance with this invention will be described below.

Process

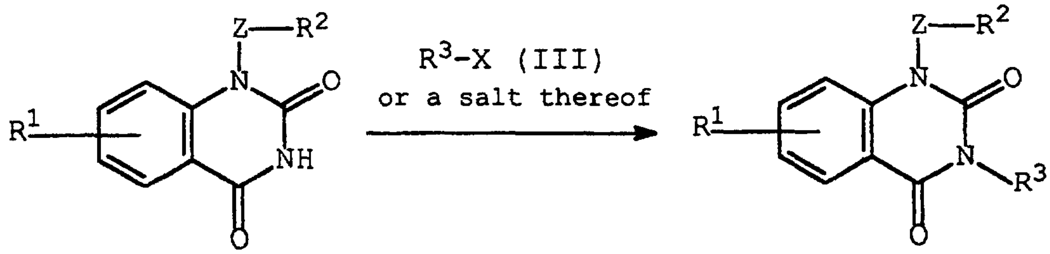


(II)
or a salt thereof

(I)
or a salt thereof

in which $R^1$, $R^2$, $R^3$ and Z are each as defined above, and X is an acid residue.

As the result of various studies in novel processes for preparation of the quinazoline derivative (I), the inventors of this invention found that the quinazoline derivative (I) was obtained safely in high yields in an unexpectedly short reaction time without variations in the reaction time by adding alkali metal carbonate to the compound (II) in a suitable solvent, by reacting the compound (III) with the mixture, and by adding a small amount of water as necessary, whereby the inventors completed this invention suited for mass synthesis.

Salts of the quinazoline derivative (I) obtained by this invention may include, for example, an alkali metal salt, such as lithium salt, sodium salt, potassium salt, etc., an alkaline earth metal salt, such as calcium salt, magnesium salt, etc.; a salt with a base, for example, a salt with an inorganic base, such as ammonium salt, etc., a salt with an organic base, such as triethylamine salt, pyridine salt, picoline salt, ethanolamine salt, triethanolamine salt, dicyclohexylamine salt, N,N'-dibenzylethylenediamine salt, etc.; a salt with an acid, for example, an inorganic acid addition salt, such as hydrochloride, hydrobromide, sulfate, phosphate, etc. and an organic acid addition salt, such as formate, acetate, trifluoroacetate, maleate, tartrate, methanesulfonate, benzenesulfonate, toluenesulfonate, etc.

The quinazoline derivative (I) and a salt thereof obtained by this invention are useful as intermediates for producing medicaments for the treatment of diabetic complication and the like.

For example, the quinazoline derivative (I) and a salt thereof are useful as intermediates for producing a quinazoline derivative represented by the following general formula (A):

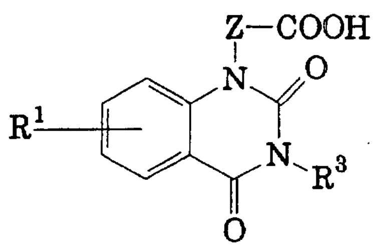

(A)

in which $R^1$, $R^3$ and Z are each as defined above.

The quinazoline derivative (A) and a salt thereof have the aldose reductase inhibitory action and are useful for the treatment and prevention of diabetic complication and the like, such as diabetic neuropathy [for example, diabetic penpheral neuropathy (for example, neuralgia, etc.), diabetic autonomic disorder (for example, impotence, etc.), etc.], diabetic nephropathy, diabetic retinopathy, diabetic keratopathy, diabetic cataract, etc. The quinazoline derivative (A) and a salt thereof can be produced by processing the quinazoline derivative (I) or a salt thereof by using a conventional method (for example, hydrolysis).

In the above and subsequent descriptions in this specification, various suitable examples included in the scopes of the definitions will be explained below in detail.

The term "lower" in this specification means, unless otherwise indicated, 1 to 6 carbon atoms.

Suitable "halogen" may include fluorine, chlorine, bromine and iodine.

Suitable "lower alkyl moiety" in "ar(lower)alkyl which may have one or more suitable substituents" may include

methyl, ethyl, propyl, isopropyl, butyl, isobutyl, tertbutyl, pentyl, hexyl and the like.

Suitable "aryl moiety" in "ar(lower)alkyl which may have one or more suitable substituents" may include phenyl, naphthyl and the like.

"Suitable substituents" in "ar(lower)alkyl which may have one or more suitable substituents" may include mono(or di or tri)halo(lower)alkyl (for example, chloromethyl, bromomethyl, chloropropyl, 1,2-dichloroethyl, 1,2-dibromoethyl, 2,2- dichloroethyl, trifluoromethyl, 1,2,2-trichloroethyl, etc.), lower alkoxy (for example, methoxy, ethoxy, propoxy, isopropoxy, butoxy, isobutoxy, pentyloxy, isopentyloxy, hexyloxy, etc.), halogen (for example, fluorine, chlorine, bromine, iodine, etc.), lower alkyl (for example, methyl, ethyl, propyl, isopropyl, butyl, isobutyl, tert-butyl, pentyl, hexyl, etc.), etc.

Suitable "protected carboxy" may include esterified carboxy, for example, lower alkoxycarbonyl (for example, methoxycarbonyl, ethoxycarbonyl, propoxycarbonyl, isopropoxycarbonyl, butoxycarbonyl, tert-butoxycarbonyl, etc.), mono (or di or tri)phenyl(lower)alkoxycarbonyl which may have nitro (for example, benzyloxycarbonyl, 4-nitrobenzyloxycarbonyl, phenethyloxycarbonyl, benzhydryloxycarbonyl, trityloxycarbonyl, etc.), in which more preferable example may be $C_1$-$C_4$ alkoxycarbonyl and the most preferable one may be ethoxycarbonyl.

Suitable "lower alkylene" may include straight or branched one, such as methylene, ethylene, trimethylene, tetramethylene, pentamethylene, hexamethylene, methyl methylene, ethyl methylene, propylene and the like, in which more preferable example may be $C_1$-$C_4$ alkylene and the most preferable one may be methylene and methyl methylene.

Suitable "acid residue" may include halogen (for example, chlorine, bromine, iodine, fluorine, etc.), or acyloxy, such as lower alkanesulfonyloxy (for example, methanesulfonyloxy, ethanesulfonyloxy, etc.), lower alkoxysulfonyloxy (for example, methoxysulfonyloxy, ethoxysulfonyloxy, etc.), arenesulfonyloxy (for example, benzensulfonyloxy, toluenesulfonyloxy, etc.), etc.).

The process for preparation of the quinazoline derivative (I) of this invention will be described below in detail.

Process

The quinazoline derivative (I) or a salt thereof can be prepared by adding alkali metal carbonate to the compound (II) or a salt thereof in a suitable solvent, by reacting the compound (III) or a salt thereof with the mixture, and by adding a small amount of water as necessary. Suitable salts of the compound (II) can be referred to the salts with acids exemplified for the compound (I). Suitable salts of the compound (III) can be referred to the salts with bases exemplified for the compound (I).

Alkali metal carbonate to be used may include potassium carbonate, sodium carbonate, etc. Preferably, pulverized potassium carbonate is used.

The reaction with the compound (II) or a salt thereof is carried out in the presence or absence of a conventional solvent which does not adversely influence the reaction, such as acetone, N,N-dimethylformamide, dichloromethane, methanol, ethanol, etc. The reaction temperature is not critical, and the reaction is usually carried out at room temperature or under heating.

By adding a small amount of water (for example, 1 to 5 weight %, preferably 2 to 4 weight %, of the compound (II) or a salt thereof) as necessary, the reaction time can be shortened without variations in the reaction time.

The process for preparation of the quinazoline derivative (I) or a salt thereof from the compound (II) or a salt thereof in accordance with this invention can be carried out continuously without being isolated and purified in the middle of the process.

It is found that the quinazoline derivative (A) and a salt thereof have the aldose reductase inhibitory action, whereby the quinazoline derivative (A) and a salt thereof are valuable as medicaments for treating diabetic complication, such as cornea injury dysraphia, cataract, neuropathy, retinopathy and nephropathy, more particularly, cataract and neuropathy.

This invention will be described below in accordance with the following Examples.

Example 1

Ethyl 2-(7-chloro-1,2,3,4-tetrahydro-2,4-dioxoquinazoline-1-yl)acetate (40 kg), acetone (200 L) and pulverized potassium carbonate (23.5 kg) were supplied into an 800 L reaction container and suspended. 4-bromo-2-fluorobenzyl bromide (49.3 kg) was added to the mixture, and reacted with the mixture for 5 hours under reflux. After glacial acetic acid (20 kg) was added to the mixture and refluxed for 2 hours, methanol (320 L) was added thereto under reflux, the mixture was cooled to 5°C or less, and crystals were obtained by filtration. The crystals were washed with methanol (80 L) and water (400 L) successively and dried under vacuum at 40°C to obtain ethyl 2-[3-(4-bromo-2-fluorobenzyl)-7-chloro-1,2,3,4-tetrahydro-2,4-dioxoquinazoline-1-yl)acetate (63.8 kg) (96% yield).

Example 2

Ethyl 2-(7-chloro-1,2,3,4-tetrahydro-2,4-dioxoquinazoline-1-yl)acetate (55 kg), acetone (385 L) and pulverized potassium carbonate (29.6 kg) were supplied into a 1500 L reaction container and suspended. 4-bromo-2-fluorobenzyl bromide (54.7 kg) was added to the mixture and water (1.65 kg) was added further and then reacted with the mixture for 1 to 2 hours at an internal temperature of 48 to 53°C. After 80% industrial acetic acid (29.2 kg) was added to the mixture and the mixture was heated for 1 to 2 hours at the same temperature, methanol (440 L) was added thereto under heating, and crystals were precipitated. The crystals were then washed with methanol (110 L) and water (550 L) successively just as in the case of Example 1 to obtain ethyl 2-[3-(4-bromo-2-fluorobenzyl)-7-chloro-1,2,3,4-tetrahydro-2,4-dioxoquinazoline-1-yl]acetate (88.6 kg) (97% yield).

Next, the obtained ethyl 2-[3-(4-bromo-2-fluorobenzyl)-7-chloro-1,2,3,4-tetrahydro-2,4-dioxoquinazoline-1-yl]acetate (88.6 kg), methanol (550 L) and water (275 L) were supplied into a 1500 L reaction container and suspended. 24% aqueous sodium hydroxide solution (27.5 L) was added to the mixture, and reaction was carried out for about 1 hour at an internal temperature of 70 to 75°C. Insolubles in the reaction solution were removed by filtration and then washed with a mixed solution of methanol (41 L) and water (41 L). After filtrate and washing were transferred to a 1500 L reaction container, 35% industrial hydrochloric acid (23.1 kg) was added dropwise thereto over about 2 hours at an internal temperature of 68 to 73°C. The temperature was then lowered to 20 to 30°C, and crystals were obtained by filtration. The crystals were washed with water (275 L) to obtain 2-[3-(4-bromo-2-fluorobenzyl)-7-chloro-1,2,3,4-tetrahydro-2,4-dioxoquinazoline-1-yl]acetic acid in a wet state.

Then, the obtained 2-[3-(4-bromo-2-fluorobenzyl)-7-chloro-1,2,3,4-tetrahydro-2,4-dioxoquinazoline-1-yl]acetic acid (wet state), 2-propanol (835 L) and water (66 L) were supplied into a 1500 L reaction container and suspended. After the mixture was dissolved at an internal temperature of 75 to 80°C, the mixture was clarified and filtered with a filter, and then washed with 2-propanol (55 L). After filtrate and washing were transferred to a 1500 L reaction container, water (127 L) was added dropwise thereto over about 30 minutes at an internal temperature of 75 to 80°C. The temperature was then lowered to 65 to 70°C, and crystals were precipitated, and stirring was carried out for about 30 minutes. After cooling to 0°C or less, the crystals were filtered. The crystals were washed with water (165 L) and dried under vacuum at 40°C to obtain 2-[3-(4-bromo-2-fluorobenzyl)-7-chloro-1,2,3,4-tetrahydro-2,4-dioxoquinazoline-1-yl] acetic acid (78.2 kg). (Yield: 91% from ethyl 2-(7-chloro-1,2,3,4-tetrahydro-2,4-dioxoquinazoline-1-yl)acetate)

Example 3

Ethyl 2-(7-chloro-1,2,3,4-tetrahydro-2,4-dioxoquinazoline-1-yl)acetate (40 kg), acetone (280 L) and pulverized potassium carbonate (21.5 kg) were supplied into a 1000 L reaction container and suspended. 4-bromo-2-fluorobenzyl bromide (38.7 kg) was added to the mixture and water (1.2 kg) was added further and then reacted with the mixture for 2 to 3 hours at an internal temperature of 45 to 55°C. After 80% industrial acetic acid (21.2 kg) was added to the mixture, and methanol (320 L) was added thereto at the same temperature. After cooling to 0°C or less, crystals were filtered. The crystals were washed with methanol (80 L) and water (400 L) successively to obtain ethyl 2-[3-(4-bromo-2-fluorobenzyl)-7-chloro-1,2,3,4-tetrahydro-2,4-dioxoquinazoline-1-yl]acetate in a wet state.

Then, the obtained 2-[3-(4-bromo-2-fluorobenzyl)-7-chloro-1,2,3,4-tetrahydro-2,4-dioxoquinazoline-1-yl]acetic acid (wet state), 2-propanol (520 L) and water (180 L) were supplied into a 1000 L reaction container and suspended. After 30% aqueous potassium hydroxide solution (27.8 kg) was added to the mixture, reaction was carried out for 1 to 2 hours at an internal temperature of 50 to 60°C. The reaction liquid was clarified and filtered with a filter, and then washed with a mixture of 2-propanol (40 L) and water (40 L). After filtrate and washing were transferred to a 1500 L reaction container, 35% industrial hydrochloric acid (16.7 kg) was added dropwise thereto over about 30 minutes at an internal temperature of 70 to 75°C, and the mixture was stirred for about 30 minutes at the same temperature. The temperature was lowered further to 5°C or less, and crystals were obtained by filtration. The crystals were washed with water (400 L) and dried under vacuum at 40°C to obtain 2-[3-(4-bromo-2-fluorobenzyl)-7-chloro-1,2,3,4-tetrahydro-2,4-dioxoquinazoline-1-yl]acetic acid (58.1 kg). (Yield: 93% from ethyl 2-(7-chloro-1,2,3,4-tetrahydro-2,4-dioxoquinazoline-1-yl)acetate)

Example 4

Ethyl 2-(7-chloro-1,2,3,4-tetrahydro-2,4-dioxoquinazoline-1-yl)acetate (25 g), acetone (175 mL) and pulverized potassium carbonate (13.4 g) were supplied into a one L reaction container and suspended. 4-bromo-2-fluorobenzyl bromide (24.9 g) was added to the mixture and water (0.75 g) was added further and then reacted with the mixture for 3 hours at an internal temperature of 45 to 55°C.

After a solution of potassium hydroxide (12.3 g) in water (150 mL) was added to the mixture, reaction was carried out for 2 hours at an internal temperature of 50 to 55°C. After acetone (75 mL) was added, 35% industrial hydrochloric

acid (31.8 g) was added at the same temperature, and water (50 mL) was added dropwise thereto. After stirring for about 30 minutes at the same temperature and cooling further to 5°C or less, crystals were filtered. The crystals were then washed with a mixture of acetone (37.5 mL) and water (37.5 mL) and water (250 mL) successively, and dried under vacuum at 40°C to obtain 2-[3-(4-bromo-2-fluorobenzyl)-7-chloro-1,2,3,4-tetrahydro-2,4-dioxoquinazoline-1-yl] acetic acid (37.5 g). (Yield: 96% from ethyl 2-(7-chloro-1,2,3,4-tetrahydro-2,4-dioxoquinazoline-1-yl)acetate)

**Claims**

1. A process for preparation of a quinazoline derivative represented by the general formula:

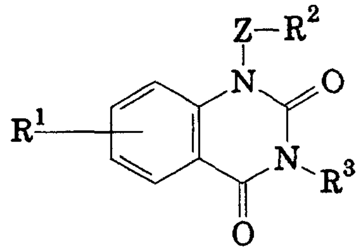

(I)

in which $R^1$ is hydrogen or halogen,

$R^2$ is protected carboxy,
$R^3$ is ar(lower)alkyl which may have one or more suitable substituents,
Z is lower alkylene,

or a salt thereof, by reacting a compound represented by the general formula:

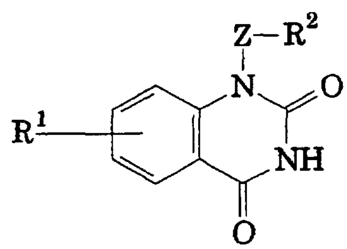

(II)

in which $R^1$, $R^2$ and Z are each as defined above,
or a salt thereof, with a compound represented by the general formula:

$$R^3\text{-}X \qquad \text{(III)}$$

in which $R^3$ is as defined above,
X is an acid residue,
or a salt thereof in a suitable solvent in the presence of alkali metal carbonate and further a small amount of water as necessary.

2. A process for preparation according to claim 1, wherein $R^1$ is halogen, $R^2$ is esterified carboxy, $R^3$ is dihalophenyl (lower)alkyl, and X is halogen.

3. A process for preparation according to claim 2, wherein $R^1$ is chloro, $R^2$ is ethoxycarbonyl, $R^3$ is 2-fluoro-4-bromobenzyl, X is bromo and Z is methylene.

4. A process for preparation according to any one of claims 1 to 3, wherein alkali metal carbonate is potassium

carbonate.

**5.** A process for preparation according to any one of claims 1 to 4, wherein 1 to 5 weight % of water is added to the compound (II) or a salt thereof.

**6.** A process for preparation according to any one of claims 1 to 4, wherein 2 to 4 weight % of water is added to the compound (II) or a salt thereof.

## INTERNATIONAL SEARCH REPORT

International application No.
PCT/JP00/01360

A. CLASSIFICATION OF SUBJECT MATTER
Int.Cl$^7$ C07D239/96

According to International Patent Classification (IPC) or to both national classification and IPC

B. FIELDS SEARCHED
Minimum documentation searched (classification system followed by classification symbols)
Int.Cl$^7$ C07D239/96

Documentation searched other than minimum documentation to the extent that such documents are included in the fields searched

Electronic data base consulted during the international search (name of data base and, where practicable, search terms used)
CAPLUS, REGISTRY, CASREACT (STN)

C. DOCUMENTS CONSIDERED TO BE RELEVANT

| Category* | Citation of document, with indication, where appropriate, of the relevant passages | Relevant to claim No. |
|---|---|---|
| X<br>Y | Shunsuke GOSHIMA et al., "1-Chikan-2,4(1H,3H) quinazorinedione no Kouritsuteki na Gousei" Chemistry Express ;vol.8(No.9) p761-764 (1993) (KINKI CHMICAL SOCIETY,JAPAN) p762; Fig-3 Chemical Abst.119:228489 | 1-4<br>5,6 |
| Y | EP, 218999, A2 (FIJISAWA PHARMACEUTICAL CO.Ltd.), 22 April, 1987 (22.04.87), page 3, Process 1,2; page 11, line 5 to page 12,line 27; EXAMPLE 6 & US,4734419,A & SU,1588283,A & JP,62-096476,A & CN,1017242,A & US,4883800,A | 1-6 |
| A | JP, 1-25767, A (Fujisawa Pharmaceutical Co., Ltd.), 27 January, 1989 (27.01.89) (Family: none) | 1-6 |
| A | M.S.MALAMAS et.al. "Quinazorineacetic Acid and Rerated Analogues as Aldose Reductase Inhibitors" Journal of Medicinal Chemistry;vol.34(No.4) p1492-1503(1991) | 1-6 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 May, 2000 (29.05.00) | 06.06.00 |
| Name and mailing address of the ISA/<br>Japanese Patent Office | Authorized officer |
| Facsimile No. | Telephone No. |

Form PCT/ISA/210 (second sheet) (July 1992)